# EUROPEAN PATENT APPLICATION

(11) **EP 4 516 895 A1**
(43) Date of publication of application: **05.03.2025**
(21) Application number: 23796349.1
(22) Date of filing: 25.04.2023
(51) Int. Cl.: C12N 5/071, C12N 5/0775, A61L 27/38

(54) **METHOD FOR PRODUCING CELL SHEET**

(30) Priority: 27.04.2022 JP 2022073877; 07.03.2023 JP 2023034995
(71) Applicant: PharmaBio Corporation, Nagoya City, Aichi 451-0025 (JP)
(72) Inventor: KUSANO, Hitoshi, Nagoya-shi, Aichi 451-0025 (JP); MIYAUCHI, Hidemasa, Nagoya-shi, Aichi 451-0025 (JP); SAKAI, Hideto, Nagoya-shi, Aichi 451-0025 (JP); YANAGAWA, Toshihide, Nagoya-shi, Aichi 451-0025 (JP); OKAZAKI, Mikiko, Nagoya-shi, Aichi 451-0025 (JP); TAZURA, Yoshiyuki, Nagoya-shi, Aichi 451-0025 (JP)
(74) Representative: Hoffmann Eitle
(86) International application number: PCT/JP2023/016201
(87) International publication number: WO 2023/210609

(57) **Abstract**

The present invention aims to provide a simple method for producing a cell sheet, which allows easy handling of the cell sheet produced by the method. The present invention provides a method for producing a cell sheet, which includes: (1) a step of preparing one or more types of mammal-derived cells; (2) a step of seeding the prepared cells on a porous membrane of a first cell culture vessel having the porous membrane on its bottom; and (3) a step of placing the first cell culture vessel in a second cell culture vessel and culturing the cells, and which allows easy handling of the cell sheet produced by the method; and provides a cell sheet produced by the method.

## Description

### [Technical Field]

The present invention relates to a method for producing cell sheets from various types of cells. In particular, the present invention relates to a method for producing cell sheets from various cells including retinal pigment epithelial cells, mesenchymal stem cells, fibroblasts, iPS cells and iPS-derived differentiated cells, ES cells and ES cell-derived final cells.

### [Background Art]

Cell culture sheet implantation therapy has been reported for the treatment of various diseases. Cell culture sheet implantation therapy is a technique in which stem cells taken from the human body or artificially produced are cultured on a plate (culture dish), and the resulting sheet-like cultured cells are placed on tissue defects caused by trauma, disease, etc. to repair the defects.

Methods of treating retinal degenerative diseases by implanting retinal pigment epithelial cells in a cell sheet state similar to the in vivo morphology have been attempted. For example, an effective treatment method for age-related macular degeneration is to implant retinal pigment epithelial cells or retinal pigment epithelium into a subretinal defect site (Patent Document 1: JP-A No. hei9-501303, Patent Document 2: JP-A No. 2008-173333). Autologous tissue implantation is performed in which a cell sheet made by cutting out retinal pigment epithelial cells (as a layer with the choroid) from the retinal tissue of a patient with age-related macular degeneration is implanted into the damaged macular area, but this cell sheet derived from the patient's tissue has problems such as an invasive risk due to a patient's retina excision surgery in addition to the implantation surgery, a high incidence of complications, and a low rate of improvement or stable maintenance of macular function after implantation.

As a method of using pigment epithelial cells such as retinal pigment epithelial cells and iris pigment epithelial cells cultured in vitro without harvesting the patient's retina, a method of using a cell sheet produced by culturing pigment epithelial cells on an artificial membrane or amniotic membrane for implantation is known. However, the composition, properties, and rigidity of the artificial membrane are different from the basement membrane created by the pigment epithelial cells themselves in vivo, and it is easy to induce inflammation and the associated rejection reaction, so it is not suitable for implantation. On the other hand, a method of easily producing a cell sheet composed of pigment epithelial cells cultured in vitro and the basement membrane created by the cells themselves has been reported (for example, Patent Document 3: WO2011/142364). The cell sheet obtained by this method has a basement membrane formed on the side opposite to the side where the cell sheet comes into contact with the planar substrate for culture, so the cell sheet can be detached from the planar substrate without the need for special cultureware such as temperature-responsive cultureware or enzymatic treatment, and in addition, the basement membrane can be brought into contact with the tissue to be implanted without turning the sheet over, simplifying the implantation procedure, and moreover, since it involves a basement membrane made of the same components as in the living body, it is easy to engraft, is rigid, and has excellent handleability, making it suitable for implantation therapy applications.

However, there is a need for a simpler and more efficient method for producing cell sheets. In addition, the produced cell sheets are detached from the culture dish and used for implantation, but since the detached cell sheets are separated from the support (flat substrate), there is a problem that the shape of the cell sheets is easily changed before implantation in the medical setting, making them difficult to handle.

In addition, a method for producing a cell sheet containing a pigment epithelial cell layer and a blood vessel forming cell layer has been reported for the purpose of supplying nutrients and oxygen to the pigment epithelium after implantation (Patent Document 4: WO2014/030749).

Mesenchymal stem cells (MSCs) are stem cells with pluripotency and self-renewal capabilities, and have been reported to have the ability to differentiate into mesoderm-derived cells such as osteoblasts, chondrocytes, adipocytes, and muscle cells, as well as the ability to differentiate into ectoderm-derived nerve cells and glial cells, and endoderm-derived hepatic cells. MSCs are also known to have homing effects, paracrine effects, and cell adhesion interactions. Based on these actions, MSCs are believed to exert the ability to repair and regenerate target tissues and cells, and the ability to regulate the immune system, such as anti-inflammation, and as a result, to show therapeutic effects on various diseases.

Cell implantation therapy using MSCs has been reported, and for example, an attempt to treat kidney disease by applying a cell sheet composition containing MSCs to the kidney has been reported (Patent Document 5: JP-A No. 2017-132744).

Methods for producing cell sheets using a variety of cells have been reported, however, there have been no reported methods for producing cell sheets that can be applied to a variety of cells, including MSCs and fibroblasts, which are cells of skin tissue, and there is a need to establish a method for producing cell sheets that can be applied to a wide range of cell types.

### [Cited References]

### [Patent Document]

[Patent Document 1] JP-A No. Hei9-501303
[Patent Document 2] JP-A No. 2008-173333
[Patent Document 3] WO 2011/142364
[Patent Document 4] WO 2014/030749
[Patent Document 5] JP-A No. 2017-132744

### [Summary of the Invention]

### [Technical Problem]

An object of the present invention is to provide a simple method for producing a cell sheet, which allows easy handling of the cell sheet produced by the method. Another object of the present invention is to provide a simple method for producing a cell sheet that can be applied to various cells.

### [Solution to Problem]

The inventors have conducted extensive research into a method for producing a cell sheet which is simple and allows easy handling of the cell sheet produced by said method, and as a result, have discovered that cells can be cultured in a cell culture vessel having a porous membrane on the bottom, thereby completing the present invention.

The present invention includes the following.
[1] A method for producing a cell sheet, comprising the steps of:
   (1) preparing one or more types of mammal-derived cells;
   (2) seeding the prepared cells on a porous membrane of a first cell culture vessel having the porous membrane on the bottom thereof; and
   (3) placing the first cell culture vessel into a second cell culture vessel, culturing cells, and producing a cell sheet on the porous membrane.
[2] The method according to the above [1], wherein at least one of the culture medium in the first cell culture vessel or the culture medium in the second cell culture vessel is a culture medium containing serum and/or a Rho kinase (ROCK) inhibitor.
[3] The method according to the above [1] or [2], wherein in the step (1), the mammal-derived cells are epithelial cells, endothelial cells, parenchymal cells or stem cells.
[4] The method according to the above [3], wherein in the step (1), the mammal-derived cells are cells selected from the group consisting of pigment epithelial cells (e.g., iris pigment epithelial cells, retinal pigment epithelial cells), fibroblasts, and mesenchymal stem cells.
[5] The method according to any one of the above [1] to [4], wherein in the step (2), the cells are seeded on the bottom at a density of 5,000 cells/mm² (preferably 10,000 cells/mm²) or more.
[6] The method according to any one of the above [1] to [5], wherein in the cell sheet obtained by culturing, a membrane containing an extracellular matrix is formed on the side of the cells opposite to the side in contact with the porous membrane.
[7] The method according to the above [6], further comprising forming tight junctions between the cells in the cell sheet obtained by culturing.
[8] The method according to the above [6] or [7], further comprising the following step (4):
   (4) a step of confirming the formation of a membrane containing an extracellular matrix on the side of the cells opposite to the side in contact with the porous membrane.
[9] The method according to any one of the above [1] to [8], further comprising the following step (5):
   (5) a step of confirming the presence or absence of the expression of a differentiation marker in the cultured cells obtained in the step (3), wherein
   when the mammal-derived cells are pigment epithelial cells such as retinal pigment epithelial cells or iris pigment epithelial cells, the molecular marker is preferably at least one selected from the group consisting of bestrophin-1, RPE-65, pan-Cytokeratin and any subtype of Cytokeratin, occludin, ZO-1, elastin, actin, type 1 collagen, type 2 collagen, and type 4 collagen, when the mammal-derived cells are epithelial cells, the molecular marker is preferably at least one selected from the group consisting of EpCAM and any subtype thereof, pan-Cytokeratin and any subtype of Cytokeratin, when the mammal-derived cells are endothelial cells, the molecular marker is preferably at least one selected from the group consisting of CD31, CD34, CD45, ICAM-1/CD54, LYVE-1, Tie2/Tek, vWF (von Willebrand factor), CD144, VCAM-1/CD106, VE cadherin and VEGF-R2, when the mammal-derived cells are fibroblasts, the molecular marker is preferably at least one selected from the group consisting of HSP47, SerpinH1, fibroblast specific protein1, CD248 (Endosialin), DDR-2, CD280 (Endo180) and Vimentin, when the mammal-derived cells are adipocytes, the molecular marker is preferably PLN1 and/or Fatty Acid Binding Protein 4, when the mammal-derived cells are skeletal muscle cells, the molecular marker is preferably Troponin t type 1 and/or myosin heavy chain, when the mammal-derived cells are smooth muscle cells, the molecular marker is preferably at least one selected from the group consisting of Transgelin, α-smooth muscle actin and Calponin, when the mammal-derived cells are cardiomyocytes, the molecular marker is preferably at least one selected from the group consisting of Fatty Acid Binding Protein 3, Cardiac Troponin T, and Nkx2.5, when the mammal-derived cells are hepatic stem cells, the molecular marker is preferably at least one selected from the group consisting of EpCAM, E-cadherin, CD133, and CD29, when the mammal-derived cells are nerve cells, the molecular marker is preferably glial fibrillary acidic protein, Myelin Basic Protein, and/or Peripherin, when the mammal-derived cells are mesenchymal stem cells, the molecular marker is preferably at least one selected from the group consisting of CD73, CD90, CD105, CD11b, CD14, CD19, CD79α, CD34, CD45, and HLA-DR, when the mammal-derived cells are T cells, the molecular marker is preferably at least one selected from the group consisting of CD3, CD4, and CD8, when the mammal-derived cells are B cells, the molecular marker is preferably at least one selected from the group consisting of CD19, CD20, CD10, CD34, CD38, CD40, and CD45R, when the mammal-derived cells are natural killer cells, the molecular marker is preferably at least one selected from the group consisting of CD16, CD56 and CD57, when the mammal-derived cells are dendritic cells, the molecular marker is preferably at least one selected from the group consisting of CD11c, HLA-DR, CD141, CD1c, CD11b, CD303, CD123 and CD1a, when the mammal-derived cells are hematopoietic stem cells, the molecular marker is preferably CD34 and/or CD45, when the mammal-derived cells are macrophages, the molecular marker is preferably at least one selected from the group consisting of CD16, CD32, CD64, CD68, CD80 and CD86; and when the mammal-derived cells are mesenchymal stem cells, in particular it is more preferable that CD73 and/or CD90 is positive and CD11b or CD14, CD19 or CD79α, CD34, CD45 and HLA-DR are negative.
[10] The method according to any one of the above [1] to [9], further comprising the following step (5'):
   (5') a step of confirming whether the cultured cells obtained in the step (3) secrete Soluble-Flt-1 (VEGF receptor 1) and/or TIMP-3.
[11] The method according to any one of the above [1] to [10], wherein in the step (1), the mammal-derived cells are pigment epithelial cells such as retinal pigment epithelial cells or iris pigment epithelial cells, mesenchymal stem cells or fibroblasts, and the cells are seeded on the bottom at 5,000 cells/mm² (preferably 10,000 cells/mm²) to 40,000 cells/mm².
[12] A cell sheet produced by the method as described in any one of the above [1] to [11].
[13] An implant material for disease treatment, comprising a cell sheet produced by the method as described in any one of the above [1] to [11].
[14] A method for producing a membrane containing extracellular matrix (e.g., a basement membrane when epithelial cells are used, and particularly, Bruch's membrane when retinal pigment epithelial cells are used), comprising a step of separating a membrane containing extracellular matrix formed on a cell sheet produced by the method as described in any one of the above [1] to [11].
[15] A cell sheet of mammal-derived cells arranged on a porous membrane, in which an extracellular matrix is present between the cells of the cell sheet, and a membrane containing the extracellular matrix (e.g., a basement membrane when epithelial cells are used, and particularly, Bruch's membrane when retinal pigment epithelial cells are used) is formed on the side opposite to the side in contact with the porous membrane.
[16] The cell sheet according to the above [15], further comprising tight junctions formed between the cells.
[17] The cell sheet according to the above [15] or [16], which is obtained by seeding one or more types of mammal-derived cells onto a porous membrane of a first cell culture vessel having the porous membrane on its bottom, and placing the first cell culture vessel in a second cell culture vessel and culturing.
[18] The cell sheet according to the above [17], wherein at least one of the culture medium in the first cell culture vessel and the culture medium in the second cell culture vessel is a culture medium containing serum and/or a Rho kinase (ROCK) inhibitor.
[19] The cell sheet according to any one of the above [15] to [18], wherein the mammal-derived cells are epithelial cells, endothelial cells, parenchymal cells or stem cells.
[20] The cell sheet according to the above [19], wherein the mammal-derived cells are cells selected from the group consisting of pigment epithelial cells (e.g., retinal pigment epithelial cells, iris pigment epithelial cells), fibroblasts, and mesenchymal stem cells.
[21] The cell sheet according to the above [20], wherein the mammal-derived cells are retinal pigment epithelial cells or iris pigment epithelial cells.
[22] The cell sheet according to any one of the above [15] to [21], wherein the cells contained in the cell sheet express at least one molecular marker selected from the group consisting of bestrophin-1, RPE-65, pan-Cytokeratin and any subtype of Cytokeratin, occludin, ZO-1, elastin, actin, type 1 collagen, type 2 collagen, and type 4 collagen.
[23] The cell sheet according to any one of the above [15] to [22], wherein the cells contained in the cell sheet secrete Soluble-Flt-1 (VEGF receptor 1) and/or TIMP-3.
[24] A method for producing a multilayered cell sheet, comprising the following steps (1) to (4):
   (1) preparing one or more types of mammal-derived cells;
   (2) seeding the prepared cells on a porous membrane of a first cell culture vessel having the porous membrane on the bottom thereof;
   (3) further placing the first cell culture vessel in a second cell culture vessel, culturing cells, and producing a cell sheet on the porous membrane; and
   (4) repeating the following steps (a) and (b) one or more times:
      (a) seeding one or more types of mammal-derived cells onto a cell sheet formed in a first cell culture vessel; and
      (b) placing the first cell culture vessel into a second cell culture vessel and culturing cells to produce a new cell sheet on the cell sheet.
[25] A multilayered cell sheet produced by the method as described in the above [24].
[26] A multilayered cell sheet arranged on a porous membrane, wherein in each cell sheet contained in the multilayered cell sheet, an extracellular matrix is present between the cells, and a membrane containing the extracellular matrix is formed on the side opposite to the side in contact with the porous membrane.

### [Advantageous Effect of the Invention]

The present invention provides a simple method for producing a cell sheet, which allows easy handling of the cell sheet produced by the method. The cell sheet obtained by the method of the present invention can be easily separated from a cell vessel by applying a physical force, for example, a water flow by pipetting, and since the cell sheet has rigidity, it is possible to deliver the cell sheet to a target implantation site by sucking or discharging the cell sheet using a thin tube or the like, without making a large incision, when implanting the cell sheet.

### [Brief Description of Drawings]

FIG. 1. It shows the results of producing cell sheets using adipose tissue-derived mesenchymal stem cells by the method of the present invention. Photographs 1 and 2 in the figure show the results of producing cell sheets by seeding and culturing 2×10⁵ cells and 10×10⁵ cells per vessel, respectively.
FIG. 2. It is a photograph showing the process of separating a membrane-like cell sheet from a cell vessel using a water flow generated by pipetting.
FIG. 3. It is a photograph showing how a cell sheet placed in physiological saline in a Petri dish was aspirated and then expelled using a pipette. The numbers indicate the order of operations.
FIG. 4. It is a photograph showing a cross section of a cell sheet formed from human adipose tissue-derived mesenchymal stem cells. The bar indicating thickness is 39.0 µm.
FIG. 5. It shows the results of immunohistochemical staining confirming the expression of each molecular marker in a cell sheet produced using adipose tissue-derived mesenchymal stem cells.

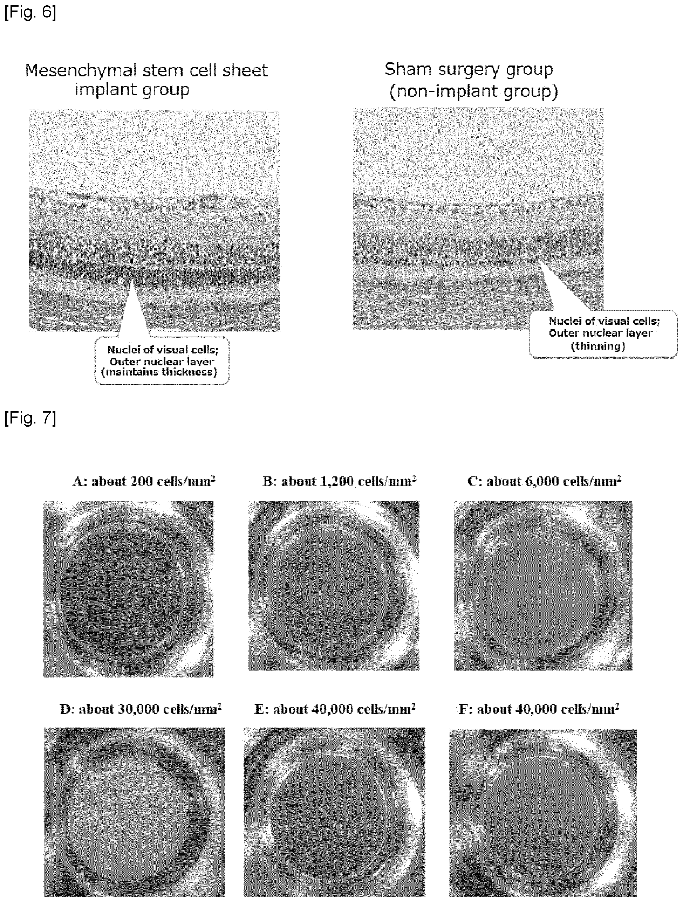

FIG. 6. It shows the results of testing the protective effect of a cell sheet formed from human adipose tissue-derived mesenchymal stem cells. The left photograph of the figure shows that the thickness of the outer nuclear layer (a layer of the nuclei of visual cells) was maintained in rats implanted with a mesenchymal stem cell sheet produced by the method of the present invention at the time point of 3 weeks after implantation. The right photograph of the figure shows that the outer nuclear layer was thinned in the control group (sham operation group) that was not implanted with a mesenchymal stem cell sheet.
FIG. 7. It shows the results of cell sheets produced using adipose tissue-derived mesenchymal stem cells by the method of the present invention. Figures A, B, C, D, E, and F are photographs showing the results of cell sheets produced by seeding 0.08×10⁵ cells, 0.4×10⁵ cells, 2×10⁵ cells, 10×10⁵ cells, 13×10⁵ cells, and 20×10⁵ cells per vessel, respectively.
FIG. 8. It shows the results of testing the phagocytic ability of a cell sheet formed from iris pigment epithelial cells by the method of the present invention. The upper row shows photographs of the results using a negative control, and the lower row shows photographs of the results using a cell sheet produced by the method of the present invention.
FIG. 9. It shows the results of immunohistochemical staining confirming that the cell sheet formed from iris pigment epithelial cells by the method of the present invention expresses elastin, type IV collagen, and type I collagen.
FIG. 10. It shows the results of testing the retinal protective effect of a cell sheet formed from iris pigment epithelial cells. The upper photograph in the figure shows that the thickness of the outer nuclear layer (a layer of the nuclei of visual cells) was maintained in rats implanted with an iris pigment epithelial cell sheet produced by the method of the present invention at the time point of 4 weeks after implantation. The lower graph in the figure shows that the photoreceptors were protected in rats implanted with an iris pigment epithelial cell sheet.
FIG. 11. It shows the results of testing the phagocytic ability of a cell sheet formed from fibroblasts.
FIG. 12. It shows that cell sheets formed from fibroblasts expressed type I collagen, type IV collagen, elastin, and ZO-1.
FIG. 13. It shows the results of a test of the retinal protective effect of a cell sheet formed from fibroblasts. The left shows the results for the fibroblast sheet implantation group, and the right shows the results for the sham operation group (control). The figure shows that 4 weeks after implantation, the thickness of the outer nuclear layer (a layer of the nuclei of visual cells) was maintained in rats implanted with the fibroblast sheet produced by the method of the present invention.
FIG. 14. It shows the results of measuring the secretion of soluble-Flt-1 (VEGF receptor 1) and TIMP-3 in cell sheets formed from human adipose tissue-derived mesenchymal stem cells.
FIG. 15. It shows the results of producing a multilayered sheet formed from human adipose tissue-derived mesenchymal stem cells by the method of the present invention.
FIG. 16. It shows the results of evaluating cell migration using cell sheets produced using mesenchymal stem cells by the method of the present invention. In each figure, the left column shows the results using a cell suspension, and the right column shows the results using a cell sheet.

### [Description of Embodiments]

Hereinafter, the present invention will be illustrated with reference to the exemplary embodiments, along with preferred methods and materials which can be used in practice of the present invention. However the present invention is not limited to the following embodiments. Unless otherwise specified in the sentences, any technical terms and scientific terms used in the present specification have the same meaning as those generally understood by those of ordinary skill in the art to which the present invention belongs. Any materials and methods equivalent or similar to those described in the present specification can be used for practicing the present invention. All publications and patents cited herein in connection with the present invention described herein are incorporated by reference, for example, as indicating methodology, materials, etc. that can be used in the present invention.

In the present specification, the notation "A-B" indicating a numerical range means a numerical range that contains the endpoints A and B. The same applies to "A to B." Furthermore, in this specification, "about" is used to mean an allowance of +10%.

The method for producing a cell sheet of the present invention includes the following steps (1) to (3) of
(1) preparing one or more types of mammal-derived cells;
(2) seeding the prepared cells on a porous membrane of a first cell culture vessel having the porous membrane on the bottom thereof; and
(3) further placing the first cell culture vessel into a second cell culture vessel, culturing cells, and producing a cell sheet on the porous membrane.

In the method of the present invention, one or more types of mammal-derived cells are prepared in the step (1), typically as a cell suspension.

The mammal-derived cells prepared in the step (1) are not particularly limited as long as they are mammal-derived cells, and examples thereof include cells derived from humans, monkeys, mice, rats, dogs, cows, horses, pigs, sheep, goats, cats, rabbits, hamsters, and guinea pigs. Preferably, the cells are human-derived.

The cell type to be prepared is not particularly limited, and may be, for example, an epithelial cell, an endothelial cell, a parenchymal cell, or a stem cell. The cell type may be either an adhesive cell or a non-adhesive cell, but is preferably an adhesive cell. Specific examples of the cell include, but are not limited to, hepatocytes, which are parenchymal cells of the liver; Kupffer cells; endothelial cells, such as vascular endothelial cells and corneal endothelial cells; fibroblasts, osteoblasts, osteoclasts, periodontal ligament-derived cells; epidermal cells, such as epidermal basal cells; epithelial cells, such as tracheal epithelial cells, digestive tract epithelial cells, cervical epithelial cells, conjunctival epithelial cells, corneal epithelial cells, iris pigment epithelial cells, and retinal pigment epithelial cells; mammary gland cells; pericytes; muscle cells, such as smooth muscle cells and cardiac muscle cells; kidney cells, pancreatic islet cells of Langerhans; nerve cells, such as peripheral nerve cells and optic nerve cells; chondrocytes; bone cells; undifferentiated stem cells, such as pluripotent stem cells, embryonic stem cells (ES cells), induced pluripotent stem cells (iPS cells), mesenchymal stem cells, hematopoietic stem cells, neural stem cells, cardiac muscle stem cells, hepatic stem cells, skeletal muscle stem cells, epithelial stem cells, epidermal stem cells, retinal stem cells, and adipose stem cells; and cells derived from these stem cells. Preferred examples include corneal endothelial cells, tracheal epithelial cells, digestive tract epithelial cells, cervical epithelial cells, corneal epithelial cells, retinal pigment epithelial cells, fibroblasts, and mesenchymal stem cells, more preferred examples include corneal epithelial cells, corneal endothelial cells, retinal pigment epithelial cells, fibroblasts, mesenchymal stem cells, iPS cells and iPS cell-derived differentiated cells, ES cells and ES cell-derived differentiated cells, and most preferred examples include retinal pigment epithelial cells, fibroblasts, and mesenchymal stem cells. Using these cells, a cell sheet can be produced by the method of the present invention.

The cells to be prepared may be primary cells directly collected from tissues or organs, or may be cells that have been passaged for several generations. Furthermore, the cells may be undifferentiated cells, such as embryonic stem cells (ES cells), pluripotent stem cells such as mesenchymal stem cells having pluripotency, induced pluripotent stem cells (iPS cells) produced from somatic cells, or stem cells including unipotent stem cells such as vascular endothelial progenitor cells having unipotency, or cells obtained by inducing the differentiation of such stem cells. The ES cells may be ES cells generated by nuclear reprogramming from somatic cells. The iPS cells are artificial stem cells derived from somatic cells that have the same characteristics as ES cells and can be produced by introducing a certain reprogramming substance (nucleic acid, protein, low molecular weight compound, etc.) into somatic cells. In addition, the target cells may be prepared by inducing differentiation of stem cells such as induced pluripotent stem cells (iPS cells) and ES cells. The conditions and medium for differentiating the stem cells into the target differentiated cells may follow the conventionally known conditions and medium, or may be appropriately set by a person skilled in the art. When the cell sheet produced by the present invention is intended for implantation, iPS cells in which histocompatibility antigens have been deleted or modified by techniques such as genome editing, or somatic cells of the subject to be implanted, can be used as the source of iPS cells. The use of iPS cells is preferable because the cell sheet obtained from these iPS cells is a cell sheet that has no antigenicity to the subject.

When the mammal-derived cells used in the step (1) of the present invention are pigment epithelial cells such as retinal pigment epithelial cells or iris pigment epithelial cells, the mammal is the same as described above, but is preferably a human. The retinal pigment epithelial cells are differentiated cells derived from stem cells or cells derived from the eyeball. Retinal pigment epithelial cells derived from the eyeball can be obtained by quickly dividing the eyeball at the equator after enucleation of a cadaver, removing the vitreous body and the retina, collecting by scraping the cells with a cell scraper or being with trypsin or EDTA solution to release them from Bruch's membrane, and then leaving them to stand in a culture medium to induce adhesion to a culture dish and proliferation, thereby proliferating a required amount of cells, and then appropriately subculturing the cells by trypsin treatment or the like to ensure the number of cells. When inducing differentiation of stem cells, human ES cells or iPS cells are cultured in an ES cell differentiation medium supplemented with Dkk-1 (Wnt antagonist) and Lefty A (Nodal antagonist). By culturing for a certain period of time, retinal progenitor cell markers Rx, Pax6, and Mitf are expressed, and human retinal pigment epithelial cells can be obtained by confirming polygonal morphology through morphological observation under an optical microscope.

When the mammal-derived cells used in the step (1) of the present invention are fibroblasts, the mammal is the same as described above, but is preferably a human. The method for preparing the fibroblast is not particularly limited, and a known method can be used. For example, the fibroblast can be prepared by separating various biological tissues by digesting it with enzymes, but is not limited to this. A non-limiting example of the biological tissue includes mammalian dermis tissue.

When the mammal-derived cells used in the step (1) of the present invention are mesenchymal stem cells, the mammal is the same as described above, but preferably human. The mesenchymal stem cells can be obtained from various tissues. Examples of tissues, which mesenchymal stem cells are derived from, include, but are not limited to, bone marrow, umbilical cord, umbilical cord blood, endometrium, placenta, amnion, chorion, decidua, dermis, dental follicle, periodontal ligament, dental pulp, tooth germ, adipose tissue, blood vessel (periphery), skeletal muscle, and synovium. The method for preparing mesenchymal stem cells is not particularly limited, and known methods can be used. For example, mesenchymal stem cells can be prepared by separating various biological tissues by digesting them with enzymes, but are not limited to this. Non-limiting examples of biological tissues include mammalian bone marrow, umbilical cord, and adipose.

As the mammal-derived cells used in the step (1) of the present invention, one or more types of cells can be used. When more than one type of cells is used, the combination is not particularly limited and can be appropriately selected within the scope of the object of the present invention. For example, cells derived from the same or different tissues, or cells derived from the same or different animal species can be used in combination. The number of cell types to be combined is not particularly limited. For example, two or more, three or more, four or more, five or more, six or more, seven or more, eight or more, nine or more, or ten or more cell types can be used in combination.

When preparing cells as a cell suspension, the suspension liquid is not particularly limited as long as it is a liquid having components that are generally used when suspending live cells. The cell suspension liquid can be prepared using a known method used in the art. In one embodiment, the cell suspension liquid may be a cell culture medium containing components for culturing cells. In one embodiment, the cell culture medium containing components for culturing cells may be added to the first cell culture vessel after the cells are seeded. In another embodiment, the cell culture medium may be added to the first cell culture vessel before the cells are seeded.

In the step (2), the prepared cells are seeded on a porous membrane of a first cell culture vessel having the porous membrane on the bottom, and a culture medium is added. If the prepared cells are a cell suspension in which the cells are suspended in a medium at a concentration suitable for culture, the culture medium does not need to be added. The shape of the first cell culture vessel is not particularly limited as long as it can hold the prepared cells and the porous membrane can be placed on the bottom. The material of the porous membrane is not particularly limited, and may be made of resin, metal, or glass.

Examples of resins that form the porous membrane include various polyethylenes such as low-density polyethylene, high-density polyethylene, and ultra-high molecular weight polyethylene, polyethylene naphthalate, polypropylene, polymethyl methacrylate, methyl methacrylate-styrene copolymer, polystyrene, fluororesins such as polytetrafluoroethylene and polyvinyl difluoride, ethylenevinyl acetate copolymer, polyamide, styrene-acrylonitrile copolymer, styrene-butadiene-acrylonitrile terpolymer, polycarbonate, polyvinyl chloride, etc. Among these, porous resin membranes formed from fluororesins such as polytetrafluoroethylene and polyvinylidene difluoride are preferred because they have excellent absorbency of the preservation solution and excellent visibility of cells under microscope observation.

Metals that form the porous membrane include copper, copper alloys, aluminum, aluminum alloys, gold, gold alloys, silver, silver alloys, tin, zinc, lead, titanium, nickel, stainless steel, etc. In addition, porous membranes made of oxides of metals such as silica, alumina, and zirconium can also be used. In addition, the porous metal and porous metal oxide membranes may be porous membranes that contain two or more of the above-mentioned metals and metal oxides.

The pore size of the porous body in the porous membrane of porous resin, porous metal, porous metal oxide, and porous glass is not particularly limited as long as it is a size that the cells used do not pass through. For example, a porous membrane having a pore size of less than 10 µm, 9 µm, 8 µm, 7 µm, 6 µm, 5 µm, 4 µm, 3 µm, 2 µm, or 1 µm can be used. In one aspect, it is preferable to have a pore size of less than 3 µm.

The porous membrane may be surface-treated to improve adhesion to adherent cells, but may not be surface-treated to facilitate the process of peeling the cell sheet of the present invention from the planar substrate. If the membrane is surface-treated, it may be surface-treated with, for example, collagen, gelatin, Matrigel, poly-L-lysine, poly-D-lysine, laminin, fibronectin, etc.

When cells are seeded on a porous membrane, the cells are seeded at a high density to suppress the shrinkage of the sheet. In this specification, the term "high density" refers to a density equal to or higher than the cell density observed in the normal tissue from which the prepared cells are derived. The upper limit can be appropriately determined by a person skilled in the art, and is, for example, a cell density at which the cells can be in close contact with each other and at which excessive seeding does not cause sheet formation defects or cell death. More specifically, the term "high density" refers to a cell density that is preferably 2.5 times or more the recommended seeding density during normal culture, and is, for example, 1 to 100 times, preferably 1.2 to 50 times, more preferably 2.5 to 30 times the cell density observed in normal tissue. The cell density observed in normal tissues varies depending on the type of tissue, but can be, for example, 1,000 cells/mm² or more, 2,000 cells/mm² or more, 3,000 cells/mm² or more, 4,000 cells/mm² or more, or 5,000 cells/mm² or more. More specifically, it is preferable that the cell density is about 3,000 cells/mm² or more for corneal endothelium, about 4,000 cells/mm² or more for retinal pigment epithelial cells, about 200 cells/mm² or more for mesenchymal stem cells, and about 5,000 cells/mm² or more for fibroblasts.

When the mammal-derived cells are retinal pigment epithelial cells, "high density" refers to "density equal to or higher than the cell density observed in a normal eyeball". Specifically, such a density is at least 4,000 cells/mm². However, even if the cell density is equal to or higher than the cell density observed in a normal eyeball, when the cells are seeded on a flat substrate at a certain density or lower, the formed cell sheet itself may be subjected to a contraction force, and the area at the time of seeding may not be maintained. This is presumably because a certain number of cells are mobilized to cover the serum contact surface of the retinal pigment epithelium, there is unevenness in the cell density, and the survival rate of the cells themselves means that the area at the time of seeding cannot be maintained. However, even this shrunken sheet can be used for the applications described below without any particular inconvenience. Therefore, the "density equal to or higher than the cell density observed in a normal eyeball" is preferably about 5,000 cells/mm² or more, more preferably about 10,000 cells/mm² or more, when the formed cell sheet is allowed to shrink from the area at the time of seeding, and is preferably about 20,000 cells/mm² or more, when the formed cell sheet is allowed to maintain the area at the time of seeding. The upper limit is a density that does not induce sheet formation failure or partial cell death due to excessive cell seeding. Taking such an upper limit into consideration, the "density equal to or higher than the cell density observed in a normal eyeball" may be preferably about 5,000 cells/mm² to about 200,000 cells/mm², more preferably about 10,000 cells/mm² to about 120,000 cells/mm², and particularly preferably about 20,000 cells/mm² to about 40,000 cells/mm².

When the mammal-derived cells are mesenchymal stem cells, "high density" refers to "density equal to or higher than the density at which cells are seeded during normal cell culture". For example, in the case of mesenchymal stem cells, the density may be, specifically, for example, about 200 cells/mm², preferably about 1,000 cells/mm², more preferably about 1,200 cells/mm², and even more preferably about 5,000 cells/mm² as the lower limit, while the upper limit may be, for example, about 200,000 cells/mm², preferably about 120,000 cells/mm², more preferably about 60,000 cells/mm², and even more preferably about 40,000 cells/mm². A preferred range may be about 1,000 cells/mm² to about 200,000 cells/mm², more preferably about 3,000 cells/mm² to about 120,000 cells/mm², even more preferably about 5,000 cells/mm² to about 60,000 cells/mm², and particularly preferably about 10,000 cells/mm² to about 40,000 cells/mm². When the mammal-derived cells are other stem cells such as iPS cells or ES cells, the "high density" is the same as the "high density" in mesenchymal stem cells.

When the mammal-derived cells are fibroblasts, the term "high density" refers to a density" equal to or higher than the density at which cells are seeded in normal cell culture". Specific examples of such a density include about 5,000 cells/mm² to about 200,000 cells/mm², more preferably about 10,000 cells/mm² to about 120,000 cells/mm², even more preferably about 10,000 cells/mm² to about 60,000 cells/mm², and particularly preferably about 20,000 cells/mm² to about 40,000 cells/mm².

In the step (3), the first cell culture vessel is placed in the second cell culture vessel. In this step, the addition of the cell culture medium to the first cell culture vessel and the second cell culture vessel can be performed at various times. For example, after the culture medium is added to the first cell culture vessel in which the cells are seeded, the first cell culture vessel may be placed in the second cell culture vessel in which the culture medium has been added in advance. In another embodiment, the first cell culture vessel in which the cells are seeded may be placed in the second cell culture vessel in which the culture medium has been added in advance, and then the culture medium may be added to the first cell culture vessel. Thereafter, the cells can be cultured to form a monolayer or multilayered cell population, and a cell sheet can be produced.

As the culture medium, any cell culture medium commonly used in the art can be used without any particular limitation. For example, depending on the type of cells used, basal media as described in Asakura Publishing Co., Ltd. "Techniques of Tissue Culture, Third Edition, edited by the Japanese Tissue Culture Association", p. 581, such as F-10 medium, F12 medium, MEM, BME medium, DMEM, αMEM, IMD medium, ES medium, DM-160 medium, Fisher medium, WE medium, RPMI1640 medium, etc., can be used. Furthermore, serum (fetal bovine serum, etc.), various growth factors, antibiotics, amino acids, etc. may be added to the above-mentioned basal medium, but preferably a medium containing serum and/or a Rho kinase (ROCK) inhibitor is used. By adding a Rho kinase (ROCK) inhibitor, apoptosis can be prevented even in high-density culture, and a good-quality cell sheet can be obtained. The pH of the medium is preferably about 6 to about 8. Culture is usually performed at about 30 to about 40°C for about 6 to about 168 hours. If necessary, the medium may be replaced and/or supplemented, or aerated or stirred.

Rho kinase (ROCK) is a serine/threonine protein kinase identified as a target protein of the small molecular weight GTP-binding protein Rho. Rock inhibitors, which are inhibitors of Rho, are commercially available and can be used without limitation in the present invention. Examples of Rock inhibitors used in the present invention include, but are not limited to, Y27632 and HA1077, and preferably HA1077. The concentration of the Rho kinase inhibitor used in the present invention can be, for example, 1 to 50 µM, preferably 2 to 30 µM, and more preferably 5 to 20 µM.

The second cell culture vessel is not particularly limited as long as it is a culture vessel for ordinary cell culture that can hold a culture medium, and examples thereof include dishes, Petri dishes, tissue culture dishes, multi-dishes, microplates, microwell plates, multi-plates, multi-well plates, chamber slides, petri dishes, trays, etc. Examples of the material of the culture vessel include, but are not limited to, inorganic materials such as metals, glass, ceramics, and silicon, and organic materials such as elastomers and plastics (e.g., polyester resins, polyethylene resins, polypropylene resins, ABS resins, nylons, acrylic resins, fluororesins, polycarbonate resins, polyurethane resins, methylpentene resins, phenolic resins, melamine resins, epoxy resins, and vinyl chloride resins).

As a combination of the first cell culture vessel having a porous membrane on the bottom and the second cell culture vessel that can be used in the method of the present invention, for example, a commercially available product can be used. For example, the first and second cell culture vessels can include a cell culture insert (the bottom of the cell culture insert is made of a porous membrane) and a culture vessel into which the insert is placed, provided by Corning International, Thermo Scientific, Greiner Bio-One International, etc. As the first cell culture vessel, for example, Corning's Transwell (registered trademark) permeable support, Snapwell (trademark) insert, Netwell (trademark) insert, Falcon cell culture insert, etc., all of which can be suitably used in the present invention. In addition, as the second cell culture vessel, for example, Corning's Falcon cell culture plate, Falcon multi-cell culture plate, etc., can be preferably used in the present invention. In the method of the present invention, since the cell-adherable surface has a porous membrane structure, it is considered to be advantageous for culture in terms of nutrition supply and oxygen supply from above and below the cell sheet, although not limited thereto. In addition, since the cell sheet produced by culture is held on the porous membrane on the bottom of the first cell culture vessel, it can be moved or transported together with the cell culture insert, and it is possible to move or transport the cells in a state in which they are stably held. Therefore, when used for implantation, the cell sheet can be peeled off from the bottom of the cell culture insert and used in the medical field where the implantation is performed, making it easy to handle.

The cell sheet obtained by the method of the present invention has an extracellular matrix, which makes the sheet robust and easy to maintain its shape. Therefore, for example, but not limited to, it can be sucked and discharged using a thin tube such as a pipette, and the sheet can be implanted to sites that are difficult to access from the body surface.

In the cell sheet obtained by the method of the present invention, a membrane containing an extracellular matrix is formed on the side of the cells opposite to the side in contact with the porous membrane. The side opposite to the side in contact with the porous membrane is the side where the cells are in contact with the culture medium. Therefore, the method of the present invention may further include the following step (4).

Step (4): A step of confirming the formation of a membrane containing an extracellular matrix on the side of the cells opposite to the side in contact with the porous membrane.

When pigment epithelial cells such as retinal pigment epithelial cells or iris pigment epithelial cells are used, the step (4) is a step of confirming the formation of Bruch's membrane. The formation of a membrane containing an extracellular matrix can be confirmed by observing the expression of elastin, type 1 collagen, type 4 collagen, and the like on the cell surface by immunostaining, or by observing protein expression by Western blotting.

In the cell sheet obtained by the method of the present invention, tight junctions can also be formed. The formation of tight junctions can be confirmed by observing the hexagonal cell morphology in close contact with each other and the expression of specific proteins between cells by immunostaining. The specific proteins expressed vary depending on the type of cells used, and can be appropriately selected with reference to publicly known information. For example, when epithelial cells are used, the formation of tight junctions can be confirmed by observing the expression of occludin, ZO-1, etc.

The method of the present invention may further include the following step (5).

Step (5): A step of confirming the presence or absence of expression of a differentiation marker in the cultured cells obtained in the step (3).

In step (5), the completion of the cell sheet can be determined by checking the presence or absence of the expression of a differentiation marker in the cultured cells obtained in the step (3). In the present specification, the differentiation marker may be expressed in any part of the cell (e.g., the cytoplasm, the cell membrane, the nuclear membrane, etc.).

As used herein, the term "differentiation marker" includes transcription products, translation products, or degradation products thereof that are specifically expressed in differentiated cells or whose expression is amplified or attenuated compared to other differentiated cells, undifferentiated cells, or progenitor cells. Examples of the differentiation marker include the following.

When the mammal-derived cells are pigment epithelial cells such as retinal pigment epithelial cells or iris pigment epithelial cells, the differentiation marker is preferably at least one selected from the group consisting of bestrophin-1, RPE-65, pan-Cytokeratin and any subtype of Cytokeratin, occludin, ZO-1, elastin, actin, type 1 collagen, type 2 collagen, and type 4 collagen.

When the mammal-derived cells are epithelial cells, the differentiation marker is preferably at least one selected from the group consisting of EpCAM and any subtype thereof, pan-Cytokeratin and any subtype of Cytokeratin.

When the mammal-derived cells are endothelial cells, the differentiation marker is preferably at least one selected from the group consisting of CD31, CD34, CD45, ICAM-1/CD54, LYVE-1, Tie2/Tek, vWF (von Willebrand factor), CD144, VCAM-1/CD106, VE cadherin, and VEGF-R2, and more preferably CD31 and/or CD34.

When the mammal-derived cells are fibroblasts, the differentiation marker is preferably at least one selected from the group consisting of HSP47, SerpinH1, fibroblast specific protein1, CD248 (Endosialin), DDR-2, CD280 (Endo180) and Vimentin.

When the mammal-derived cells are adipocytes, the differentiation marker is preferably PLN1 and/or Fatty Acid Binding Protein 4.

When the mammal-derived cells are skeletal muscle cells, the differentiation marker is preferably Troponin t type 1 and/or myosin heavy chain.

When the mammal-derived cells are smooth muscle cells, the differentiation marker is preferably at least one selected from the group consisting of Transgelin, α-smooth muscle actin, and Calponin.

When the mammal-derived cells are cardiomyocytes, the differentiation marker is preferably at least one selected from the group consisting of Fatty Acid Binding Protein 3, Cardiac Troponin T, and Nkx2.5.

When the mammal-derived cells are liver cells, the differentiation marker is preferably at least one selected from the group consisting of EpCAM, E-cadherin, CD133 and CD29.

When the mammal-derived cells are nerve cells, the differentiation marker is preferably glial fibrillary acidic protein, myelin basic protein and/or peripherin.

When the mammal-derived cells are T cells, the differentiation marker is preferably at least one selected from the group consisting of CD3, CD4 and CD8.

When the mammal-derived cells are B cells, the differentiation marker is preferably at least one selected from the group consisting of CD19, CD20, CD10, CD34, CD38, CD40 and CD45R.

When the mammal-derived cells are natural killer cells, the differentiation marker is preferably at least one selected from the group consisting of CD16, CD56 and CD57.

When the mammal-derived cells are dendritic cells, the differentiation marker is preferably at least one selected from the group consisting of CD11c, HLA-DR, CD141, CD1c, CD11b, CD303, CD123 and CD1a.

When the mammal-derived cells are hematopoietic stem cells, the differentiation marker is preferably CD34 and/or CD45.

When the mammal-derived cells are macrophages, the differentiation marker is preferably at least one selected from the group consisting of CD16, CD32, CD64, CD68, CD80 and CD86.

When the mammal-derived cells are mesenchymal stem cells, the differentiation marker is preferably at least one selected from the group consisting of CD73, CD90, CD105, CD11b, CD14, CD19, CD79α, CD34, CD45, and HLA-DR. In particular, it is more preferable that CD73 and/or CD90 is positive, and CD11b or CD14, CD19 or CD79α, CD34, CD45, and HLA-DR are negative.

When the mammal-derived cells are iPS cell-derived differentiated cells or ES cell-derived differentiated cells, the differentiation marker can be appropriately selected depending on the type of differentiated cells to be produced or the intended use of the cell sheet.

In addition, examples of nerve cell differentiation markers include tubulin (especially β-tubulin), MAP2, neurofilament, and neuron-specific enolase, examples of adipocyte differentiation markers include aP2, glycerophosphate dehydrogenase, adipsin, and leptin, and examples of osteoblast differentiation markers include procollagen 1α1, RUNX2, alkaline phosphatase, osteopontin, and osteocalcin.

"Confirming the presence or absence of expression of a differentiation marker" is not particularly limited as long as it is a sample derived from the cells cultured in the step (3) and contains a differentiation marker (e.g., RNA, protein, its degradation products, etc.).

When the sample is a cell, the cells cultured in the step (3) can be subjected to immunostaining, and the expression level of the differentiation marker can be measured, for example, by flow cytometry.

When the sample is RNA, the expression of the differentiation marker gene can be examined by preparing an RNA (e.g., total RNA, mRNA) fraction from the cells cultured in the step (3) and detecting the transcription product of the marker gene contained in the fraction, or by directly detecting the marker gene product in the cells without extracting RNA from the cells. Preparation of an RNA (e.g., total RNA, mRNA) fraction from the cells can be performed according to a conventional method, for example, using a commercially available RNA extraction kit. Examples of means for detecting the transcription product of the differentiation marker gene in the RNA fraction include methods using hybridization (Northern blot, dot blot, DNA chip analysis, etc.) or methods using PCR (RT-PCR, competitive PCR, real-time PCR, etc.), with quantitative PCR or DNA chip analysis being preferred. When detecting the marker gene without extracting RNA from the cells, in Situ Hybridization can be used as the detection means, and the expression of a differentiation marker can be directly confirmed on the serum-contacting surface of cells, and therefore the method is preferably used in the present invention.

Alternatively, the expression of the differentiation marker gene can be confirmed by preparing a protein fraction from the cells and detecting the translation product of the marker gene contained in the fraction (i.e., marker protein), or by directly detecting the translation product of the marker gene in the cells without extracting protein from the cells. The detection of the marker protein can be performed by immunological measurement (e.g., ELISA, FIA, RIA, Western blot, etc.) using an antibody against each protein, or, for proteins that exhibit measurable physiological activity such as enzymes, the physiological activity can be measured using a known method for each marker protein. Alternatively, the detection of the marker protein can be performed using mass spectrometry such as MALDI-TOFMS.

In a preferred embodiment, cells contained in the cell sheet produced by the method of the present invention can secrete a factor that suppresses the development of neovascularization. For example, but not limited to, a cell sheet produced from iris pigment epithelial cells by the method of the present invention can secrete soluble-Flt-1 (VEGF receptor 1) 1 and TIMP-3, which are factors that suppress the development of choroidal neovascularization.

In another preferred embodiment, the cells contained in the cell sheet produced by the method of the present invention are less likely to migrate, and cell detachment from the cell sheet due to cell migration is hardly observed.

The cell sheet produced by the method of the present invention has rigidity, and cells are less likely to migrate from the cell sheet. The cell sheet of the present invention can be applied to local areas in the body where administration of cell suspensions has been used up until now, and is less likely to cell migration, which is a problem with suspensions. Therefore, the cell sheet of the present invention can be used for target diseases where local administration is preferable.

Hereinafter, an embodiment in which a cell sheet is produced by the method of the present invention using pigment epithelial cells such as retinal pigment epithelial cells or iris pigment epithelial cells will be described, but the present invention is not limited thereto.

When a cell sheet is produced by the method of the present invention using retinal pigment epithelial cells, the membrane containing extracellular matrix in the above step (4) is, in one embodiment, Bruch's membrane. "Bruch's membrane" is a thin membrane between the retinal pigment epithelium and the choroid, and is a layer that supports the retinal pigment epithelium. It is an acellular structure mainly composed of collagen fibers, to which the choroid and pigment epithelial cells adhere, and serves as a passageway for sending substances from the choroid to the outer layer of the retina, which has no blood vessels. In the method of the present invention, a membrane containing an extracellular matrix made by the cells themselves, such as Bruch's membrane containing elastic fibers and basement membrane, is formed, thereby obtaining a strength that can withstand implantation operations. In addition, in the method of the present invention, the membrane containing extracellular matrix can be formed on the side of the cells opposite to the side that contacts the porous membrane, so that the cell sheet can be easily separated from the first cell culture vessel together with the membrane containing extracellular matrix.

The present invention also provides an in vitro method for producing a membrane containing an extracellular matrix, characterized in that a membrane containing an extracellular matrix formed in the method for producing a cell sheet is separated from the cell sheet. The membrane containing the extracellular matrix formed on the retinal pigment epithelium can be peeled off and collected from the sheet by EDTA treatment, or by pipetting and suctioning the sheet surface in a magnesium-and calcium-free medium. The membrane can also be peeled off and collected by suctioning the culture medium and exposing it to air, or by dehydrating the sheet surface by dilute alcohol treatment.

The present invention also relates to a cell sheet obtained according to the cell sheet production method, preferably a pigment epithelial cell sheet such as a retinal pigment epithelial cell sheet or an iris pigment epithelial cell sheet. The cell sheet of the present invention can be used in various applications in biological objects, such as screening applications and toxicity testing applications. Furthermore, the cell sheet of the present invention can be suitably implanted as an implant material for disease treatment to a subject in need of tissue implantation. In particular, the retinal pigment epithelial cell sheet of the present invention is suitable as an implant material for retinal treatment for patients with ocular diseases. Examples of ocular diseases include age-related macular degeneration, retinitis pigmentosa, and retinal pigment streaks. Furthermore, the retinal pigment epithelial cell sheet of the present invention can be used for various screening applications such as drug efficacy screening and toxicity evaluation for the ocular diseases. For example, the cell sheet of the present invention can be applied to screening of toxic substances according to the method described in JP-A-2007-517210. Furthermore, the retinal pigment epithelial cell sheet of the present invention can be used as an in vitro model for evaluating various functions of retinal pigment epithelial cells in the living body, such as functions related to the maintenance of visual cells, such as the neuroprotective action and phagocytic ability of visual cell outer segments, retinal vascular barrier functions by pump action, and tight junctions.

The present invention also relates to a cell sheet, preferably a retinal pigment epithelial cell sheet, in a state held on a porous membrane, obtained according to the cell sheet production method. Since the produced cell sheet is held (fixed) on the membrane, it is possible to prevent shape changes such as shrink, inversion, curling, etc. Furthermore, since the cell sheet is held on the membrane, it is possible to prevent the shape changes even if physical stimuli such as movement or transportation are applied, and the cell sheet can be implanted in a good condition.

The implant material for disease treatment of the present invention can be used to treat diseases in humans and non-human mammals (e.g., monkeys, mice, rats, dogs, cows, horses, pigs, sheep, goats, cats, rabbits, hamsters, guinea pigs, etc.).

The range of the diseased area to which the implantation material for disease treatment of the present invention can be applied varies depending on the target disease, the animal species, age, sex, weight, symptoms, etc. of the recipient animal, and for example, when applied to age-related macular degeneration, the range of the diseased area to be implanted is usually in the range of 0.07 cm² to 0.28 cm².

The implantation material for disease treatment of the present invention may be implanted at once or in several separate batches. The number of implants is determined by medical professionals and guidelines depending on the disease, but for example, when the disease is age-related macular degeneration, the retinal pigment epithelial cell sheet of the present invention may be implanted two or more times depending on the severity. When implanting multiple times, the interval between implants is not particularly limited, but may be several days to several weeks.

The implantation material for disease treatment of the present invention is implanted by a medical professional according to an appropriate implantation method in accordance with the guidelines. For example, for treatment sites in the thorax or abdominal cavity, thoracotomy or laparotomy can be applied, and endoscopic techniques can also be adopted. When implanting a retinal pigment epithelial cell sheet as the implantation material for disease treatment of the present invention subretina, it can be implanted by using a water flow from a syringe needle inserted to the implantation site under the retina of the eye, or by using a dedicated transport treatment device.

The present invention further provides a membrane containing an extracellular matrix obtained by the method for producing a membrane containing an extracellular matrix. Such a membrane can be obtained by preparing a cell sheet using the method of the present invention, and then removing the cells alternatively, in addition to peeling the membrane from the sheet. The cells can be removed by, for example, freeze-drying or freezing and thawing. Therefore, the method for producing a membrane containing an extracellular matrix of the present invention may further include a step of removing the cells by a freeze-drying step to obtain a membrane in addition to the above-mentioned method for producing a cell sheet of the present invention. Since the membrane containing an extracellular matrix of the present invention is a basement membrane that does not contain cells, there are fewer regulatory restrictions in implanting the membrane than in implanting cells, and the hurdle for practical use is lower. It is also useful for research applications such as functional analysis.

The above describes the embodiment of producing a cell sheet by the method of the present invention using retinal pigment epithelial cells, but when a different cell type is used as the mammal-derived cell, it is clear to a person skilled in the art that the administration target, target disease, implantation method, etc. are appropriately changed according to the cell used, referring to guidelines, etc. For example, when fibroblasts are used as the mammal-derived cell, the target disease can be, but is not limited to, skin-related diseases. Furthermore, when mesenchymal stem cells are used as the mammal-derived cell, the target disease can be, but is not limited to, retinal pigment epithelial degeneration disease and nerve damage disease.

In one aspect, the present invention provides a multilayered cell sheet comprising a plurality of cell sheets and a method for producing the same. A multilayered cell sheet can be produced by repeating the process of seeding cells again on cells that have already been formed into a sheet by the above-mentioned cell sheet production method and forming another cell sheet. Each cell sheet contained in the multilayered cell sheet may be a cell sheet containing a single cell type, or may be a cell sheet containing multiple cell types. Each cell sheet contained in the multilayered cell sheet may contain different cell types for each cell sheet, or may contain the same cell type.

By combining cell sheets made of cells with different functions, or by layering cell sheets containing cells with different functions, it is possible to form artificial tissues (Allergy, 2013, vol. 62, pp. 25-32). For example, by forming a cell sheet or multilayered cell sheet using a cell suspension in which stromal cells or mesenchymal stem cells are mixed with antigen-presenting cells, hematopoietic cells such as lymphocytes, or hematopoietic stem cells, it is possible to produce artificial lymphoid tissue or artificial bone marrow. Such multilayered cell sheets can be used to treat immune deficiency, severe infections, and malignant tumors, as well as hematopoietic insufficiency.

By forming mesenchymal stem cells into a sheet, the expression of the marker CD105 is reduced or eliminated, and a CD105-negative mesenchymal subset with strong immunosuppressive properties can be induced. Such a multilayered cell sheet formed by layering mesenchymal stem cell sheets can be used for preventing or suppressing rejection after organ implantation, preventing or suppressing rejection during allogeneic mesenchymal stem cell implantation, treating autoimmune diseases, etc.

Therefore, in one aspect, the present invention provides a method for producing a multilayered cell sheet, comprising the following steps (1) to (4) of:
(1) preparing one or more types of mammal-derived cells;
(2) seeding the prepared cells on a porous membrane of a first cell culture vessel having the porous membrane on the bottom thereof;
(3) placing the first cell culture vessel in a second cell culture vessel, culturing cells, and producing a cell sheet on the porous membrane;
(4) repeating the following steps (a) and (b) one or more times:
   (a) seeding one or more types of mammal-derived cells onto a cell sheet formed in a first cell culture vessel;
   (b) placing the first cell culture vessel into a second cell culture vessel and culturing cells to produce a new cell sheet on the cell sheet;

In one aspect, the present invention provides a multilayered cell sheet produced by the above-mentioned method, and an implant material for disease treatment comprising the multilayered cell sheet.

In one aspect, the present invention provides a multilayered cell sheet arranged on a porous membrane, in which an extracellular matrix is present between the cells of each cell sheet included in the multilayered cell sheet, and a membrane containing the extracellular matrix is formed on the side opposite to the side in contact with the porous membrane. Note that in the multilayered cell sheet, the membrane containing the extracellular matrix may be formed between each layer in addition to on the side opposite to the side in contact with the porous membrane of the cell sheet.

### [Examples]

The present invention will be specifically described below with reference to examples, but the present invention is not limited to the following examples.

### Example 1

Formation of human adipose tissue-derived mesenchymal stem cell sheets

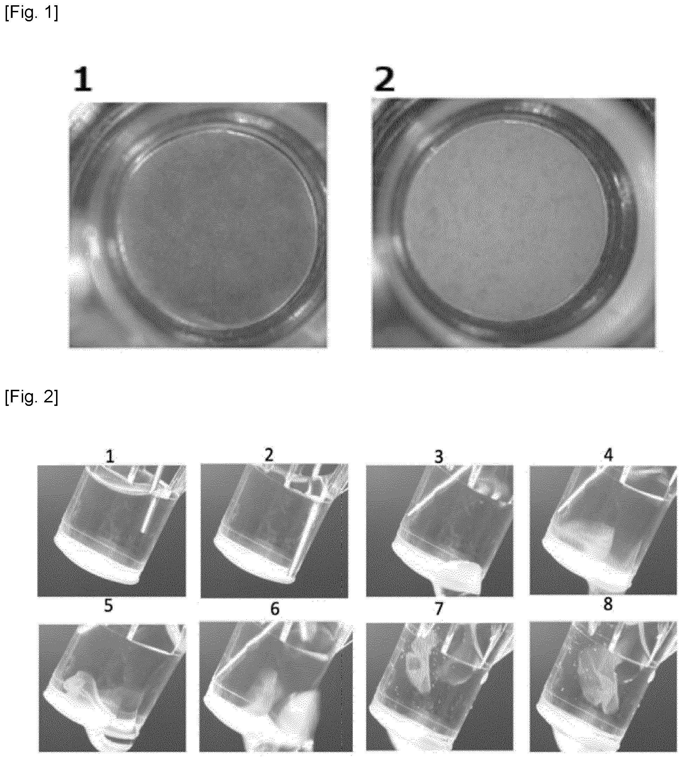

Human adipose tissue-derived mesenchymal stem cells (PromoCell GmbH Sickingenstr. 63/6569126 Heidelberg Germany) obtained from human tissue were suspended in a culture medium for mesenchymal stem cells. The produced cell suspension was added onto the porous membrane (diameter 6.5 mm Transwell with 0.4 µm pore polyester membrane) on the bottom of the cell culture insert, and cells were seeded at 2.0×10⁵ cells or 10×10⁵ cells per vessel. The cell culture insert was immersed in the well filled with culture medium in advance, and cultured, and the formation of a cell sheet was confirmed (FIG. 1). The production of a cell sheet was confirmed for all cell numbers. 2.0×10⁵ cells/vessel (bottom diameter 6.5 mm) corresponds to approximately 6,000 cells/mm² bottom area. It was observed that by seeding cells at such a high density, a cell sheet without shrink could be obtained. Therefore, in the following experiments, cells were seeded at a cell number of 2×10⁵ cells or more per vessel to produce a cell sheet.

When the obtained cell sheet was observed under a microscope, it was observed that a membrane containing extracellular matrix was formed on the side opposite to the porous membrane, and the cell sheet could be easily separated from the cell vessel by physically applying a water flow by pipetting. FIG. 2 shows how the membrane-like cell sheet was separated from the cell vessel by pipetting a water flow.

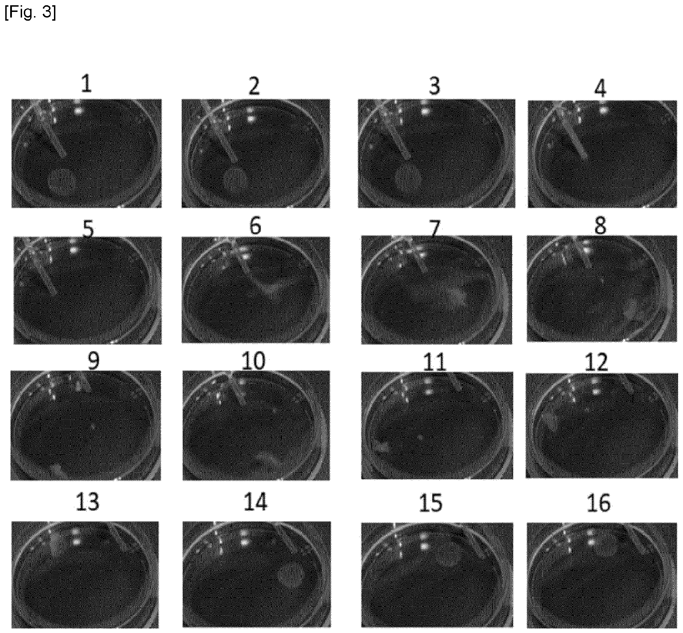

The cell sheet separated from the cell vessel by pipetting was transferred to a Petri dish containing physiological saline. The cell sheet was aspirated by using a pipette in the Petri dish, and then expelled. It was confirmed that the cell sheet maintained its shape and had rigidity, and that the cell sheet was easy to move when implanted. The state of pipetting is shown in FIG. 3.

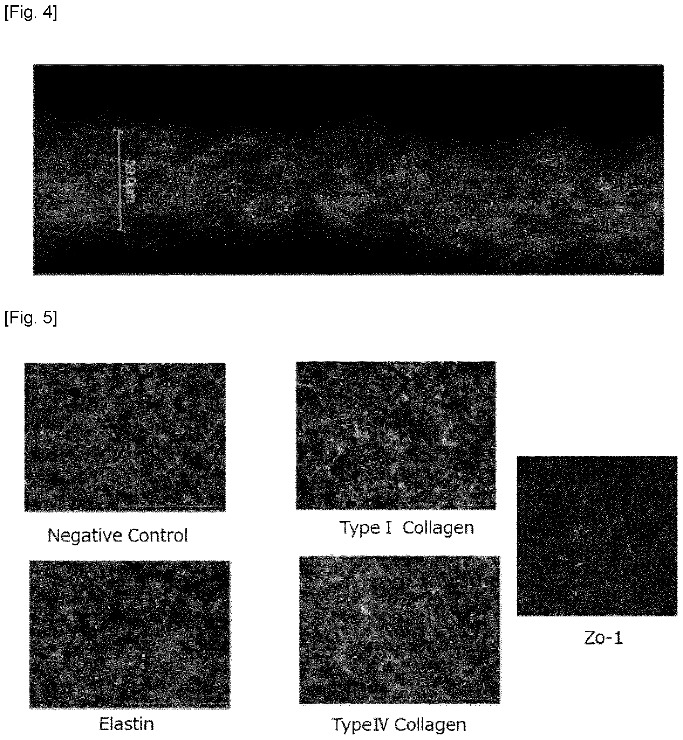

The cell sheet separated from the cell vessel was fixed with 10% paraformaldehyde, and a 14-micrometer-thick frozen section was produced and nuclear-stained with DAPI. As a result, the obtained cell sheet was found to be 20-40 micrometer-thick (FIG. 4).

The cell sheet separated from the cell vessel was stored at 2-8°C for 96 hours, and then cultured for 48 hours. PEDF, which is involved in protecting visual cells, and VEGF, which is essential for maintaining choriocapillaries, secreted from the cell sheet in said culturing procedure, were measured by ELISA. The results are shown in Table 1 below.

**[Table 1]**

| **Secretion measurement by ELISA** | |
|---|---|
| **Cytokine** | **Concentration (ng/mL)** |
| **Vascular endothelial growth factor (VEGF)** | **3.73~11.13** |
| **Pigment epithelium-derived factor (PEDF)** | **215.5~641.5** |

Furthermore, the expression of each molecular marker was measured by immunohistochemical staining according to a standard method. It was confirmed that the mesenchymal stem cell sheet produced by the method of the present invention expressed elastin, type IV collagen, type I collagen, and Zo-1 (FIG. 5).

Furthermore, when the produced mesenchymal stem cell sheet was implanted into retinal degeneration rats (Royal College Surgeon [RCS] rats), it was confirmed that a retinal protective effect was obtained. In observations three weeks after implantation, the thickness of the outer nuclear layer (the layer in which the nuclei of visual cells are arranged) was maintained in rats implanted with the mesenchymal stem cell sheet produced by the method of the present invention (left photograph in FIG. 6). On the other hand, in disease-developed rats not implanted with a cell sheet, the outer nuclear layer was thinned (right photograph in FIG. 6). When the thickness of the outer nuclear layer (i.e., the degree of visual cell protection) was compared between the treated eye (right) and the untreated eye (left) and scored, a statistically significant difference (p<0.01, Wilcoxon rank sum test) was observed between the mesenchymal stem cell sheet implant group (n=10) and the sham group (n=5).

Furthermore, as shown in Table 2 below, photoreceptors were protected in rats implanted with the mesenchymal stem cell sheet produced by the method of the present invention. It was confirmed that implantation of the mesenchymal stem cell sheet produced by the method of the present invention has the effect of suppressing the progression of lesions and preserving visual cells.

**[Table 2]**

| **Test group** | **Sham** | | | | | **Mesenchymal stem cell sheet implantation** | | | | |
|---|---|---|---|---|---|---|---|---|---|---|
| **Findings Grade** | **-** | **±** | **+** | **2+** | **3+** | **-** | **±** | **±** | **2+** | **3+** |
| **Ocular** | | | | | | | | | | |
| **Protection of photoreceptor cell** | **5** | **0** | **0** | **0** | **0** | **2** | **5** | **3** | **0** | **0** |
| **Grade : -: no abnormal change, +: very slight, +: slight, 2+: moderate, 3+: severe** | | | | | | | | | | |

As a safety test of the mesenchymal stem cell sheet produced by the method of the present invention, a general toxicity test (GLP test) was conducted using immunodeficient rats, and no graft failure or early dropout was observed, and no adverse events were observed.

### Example 2: Examination of the number of cells to be seeded

In the same manner as in Example 1, cell sheets were produced using human adipose tissue-derived mesenchymal stem cells. A cell suspension was added to a vessel having a porous membrane with a diameter of 6.5 mm on the bottom so that the following cell numbers were obtained. The number of cells seeded per vessel was 0.08×10⁵ cells, 0.4×10⁵ cells, 2.0×10⁵ cells, 10×10⁵ cells, 13×10⁵ cells, and 20×10⁵ cells. The cell concentrations per bottom area of the vessel correspond to approximately 240 cells/mm² bottom area, approximately 1200 cells/mm² bottom area, approximately 6,000 cells/mm² bottom area, 30,000 cells/mm² bottom area, approximately 40,000 cells/mm² bottom area, and approximately 60,000 cells/mm² bottom area, respectively. The formation of cell sheets was confirmed at all cell concentrations. The results are shown in FIG. 7.

### Example 3

### Formation of iris pigment epithelial cell sheets

A cell sheet was produced in the same manner as in Example 1 using iris pigment epithelial cells obtained from human iris tissue.

In vitro tests showed that the obtained iris pigment epithelial cell sheets exhibited (1) phagocytic ability and (2) secretion of PEDF and VEGF, which are characteristic of normal retinal pigment epithelial cells, and (3) possessed an extracellular matrix-containing membrane structure that functioned as a scaffold for cell regeneration, similar to Bruch's membrane.

The cell sheet was stored at 2-8°C for 96 hours and then cultured for 48 hours. PEDF, which is involved in protecting visual cells, and VEGF, which is essential for maintaining choriocapillaries, secreted from the cell sheet in said culturing procedure, were measured by ELISA, and the results are shown in Table 3 below.

**[Table 3]**

| **Secretion measurement by ELISA** | |
|---|---|
| **Cytokine** | **Concentration (ng/iinL)** |
| **Vascular endothelial growth factor (VEGF)** | **2.1 ~ 18.3** |
| **Pigment epithelium-derived factor (PEDF)** | **1.4 ~ 58.4** |

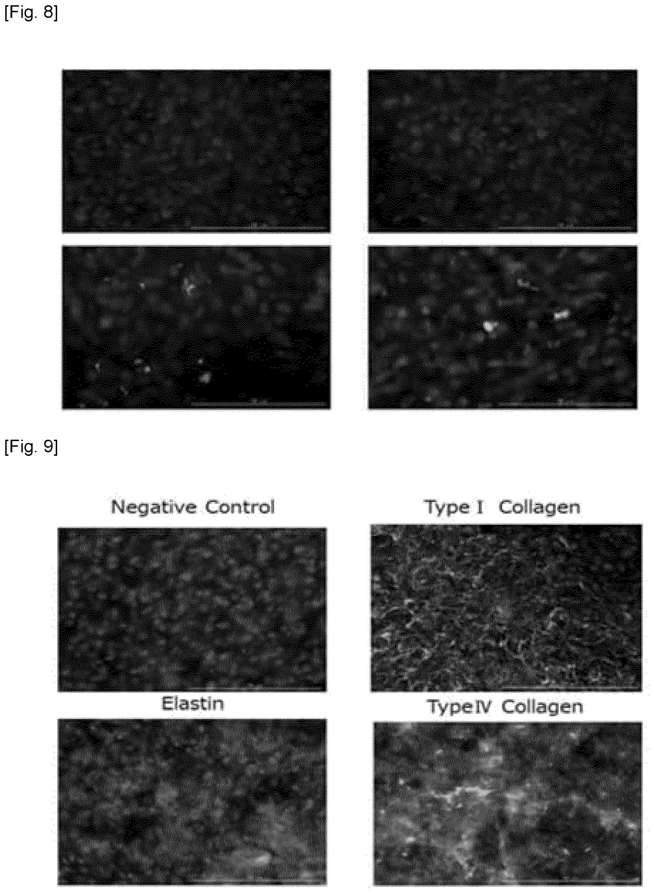

Data showing the phagocytic ability of the cell sheet cultured in the same manner are shown in FIG. 8. The phagocytic ability test was performed using a Phagocytosis Assay Kit from Cayman Chemical (US). The test was performed by mixing the cell sheet with fluorescently labeled latex beads, incubating, washing with trypan blue, and confirming the phagocytosed latex beads by fluorescent microscopy. A sheet reacted with a fluorescently labeled secondary antibody that was not conjugated to the beads was used as a negative control. In FIG. 8, the top two photographs show the data of the negative control, and the bottom two photographs show the data of the cell sheet produced by the method of the present invention.

The expression of each molecular marker was confirmed according to a standard method. It was confirmed that the retinal pigment epithelial cell sheet produced by the method of the present invention expressed elastin, type IV collagen, and type I collagen (FIG. 9).

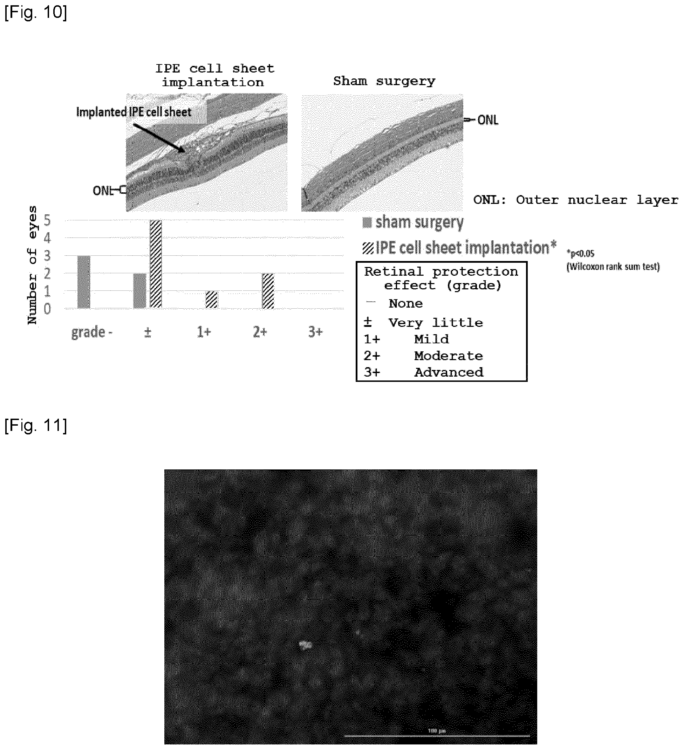

When the iris pigment epithelial cell sheet produced by the method of the present invention was implanted into retinal degeneration rats (RCS rats), it was confirmed that a retinal protective effect was obtained (FIG. 10). Four weeks after implantation, the thickness of the outer nuclear layer (a layer of the nuclei of visual cells) was maintained in the rats implanted with the retinal pigment epithelial cell sheet produced by the method of the present invention (upper photograph in FIG. 10). Furthermore, the photoreceptors were protected in the rats implanted with the retinal pigment epithelial cell sheet produced by the method of the present invention (lower graph in FIG. 10).

As a safety test of the iris pigment epithelial cell sheet produced by the method of the present invention, a general toxicity test (GLP test) was conducted using immunodeficient rats, and no graft failure or early dropout was observed, nor were any adverse events observed. Furthermore, the implantation method, which is considered to be the biggest problem with sheet-type products, has already been established through repeated trials using primates.

### Example 4

### Fibroblast sheet formation

A cell sheet was produced in the same manner as in Example 1 using fibroblasts obtained from human tissue.

In vitro tests showed that the obtained fibroblast sheets exhibited (1) phagocytic ability and (2) secretion of PEDF and VEGF, which are characteristic of normal retinal pigment epithelial cells, and (3) possession of an extracellular matrix-containing membrane structure that functioned as a scaffold for cell regeneration, similar to Bruch's membrane.

The cell sheet was stored at 2-8°C for 96 hours and then cultured for 48 hours. PEDF and VEGF secreted from the cell sheet in said culturing procedure were measured by ELISA, and the results are shown in Table 4 below, and similarly, data showing that the cultured cell sheet has phagocytic ability is shown in FIG. 11.

**[Table 4]**

| **Secretion measurement by ELISA** | |
|---|---|
| **Cytokine** | **Concentration (ng/mL)** |
| **Vascular endothelial growth factor (VEGF)** | **16.8~45.9** |
| **Pigment epithelium-derived factor (PEDF)** | **45.1~213.3** |

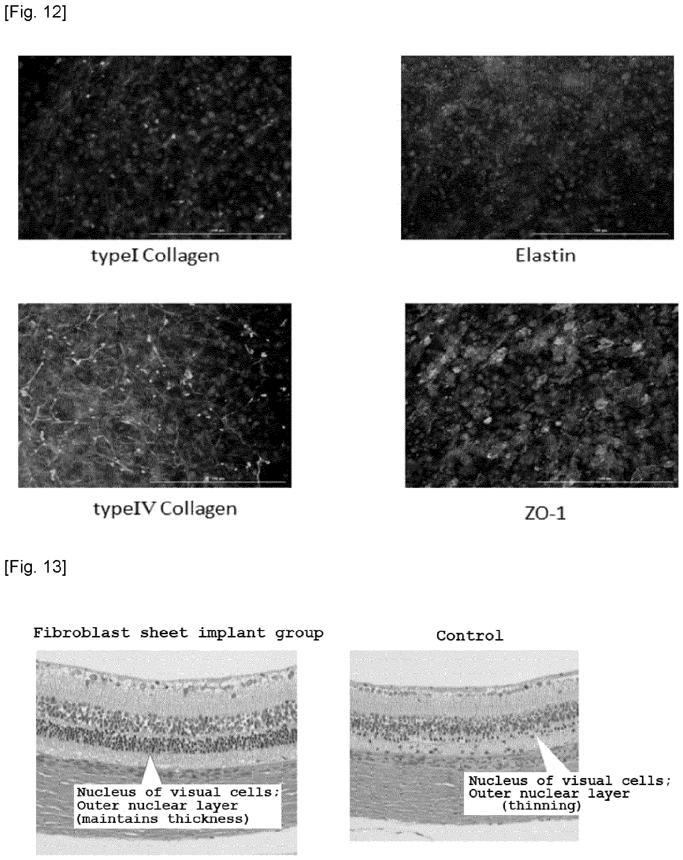

The expression of each molecular marker was measured by immunohistochemical staining according to a conventional method. The fibroblast sheet produced by the method of the present invention expressed type I collagen, type IV collagen, elastin, and ZO-1 (FIG. 12).

When the fibroblast retinal pigment epithelial cell sheet produced by the method of the present invention was implanted into retinal degeneration rats (RCS rats), it was confirmed that the sheet had a retinal protective effect (Table 5). Four weeks after implantation, significant protection from thinning of the outer nuclear layer (a layer of the nuclei of visual cells) (maintenance of thickness) was confirmed (FIG. 13).

**[Table 5]**

| **Test group** | **Sham** | | | | | **Fibroblast sheet** | | | | |
|---|---|---|---|---|---|---|---|---|---|---|
| **Findings Grade** | **-** | **±** | **±** | **2+** | **3+** | **-** | **±** | **±** | **2+** | **3+** |
| **Ocular** | | | | | | | | | | |
| **Protection of photoreceptor cell** | **5** | **0** | **0** | **0** | **0** | **0** | **3** | **6** | **1** | **0** |
| **Grade ; -⁻: no abnormal change. ±: very slight. +: slight. 2+: moderate. 3+: severe** | | | | | | | | | | |

The purity of the target cells in the cell sheets was determined by checking the expression state of CD90, Vimentin, and CK18. As a result, as shown in Table 6 below, it was confirmed that the target cell purity was maintained at a high level in the cell sheets subjected to the toxicity test and efficacy test. It was also confirmed that a relatively high level of purity was maintained in the samples after transportation.

**[Table 6]**

| **Isotype criteria** | **CD90** | **Vimentin** | **CK18** |
|---|---|---|---|
| **Toxicity test 1 sample** | **98.04%** | **98.16%** | **0.00%** |
| **Toxicity test 2 sample** | **97.72%** | **92.95%** | **0.14%** |
| **Drug efficacy test sample** | **99.87%** | **98.60%** | **0.10%** |
| **Post-transport sample** | **88.29%** | **85.11%** | **11.71%** |

As a safety test of the fibroblast sheet produced by the method of the present invention, a general toxicity test (GLP test) was conducted using immunodeficient mice, and no graft failure or early dropout was observed, and no adverse events were also observed.

### Example 5

### Confirmation of secretion of angiogenesis inhibitors from mesenchymal stem cell sheets

A cell sheet was produced using human adipose tissue-derived mesenchymal stem cells obtained from human tissue in the same manner as in Example 1. As a comparative example, plate culture was performed using human adipose tissue-derived mesenchymal stem cells obtained from human tissue. In the plate culture, cells were seeded in a resin cell culture vessel at an optimal density according to the culture area, and cultured by adhering them to the culture surface.

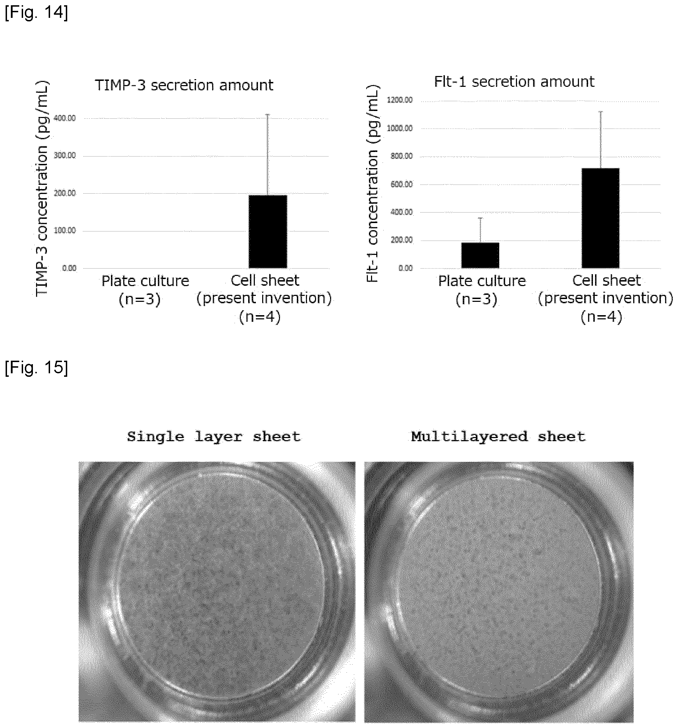

After the culture, soluble-Flt-1 (soluble VEGF receptor 1) and TIMP-3 secreted into the medium were measured by ELISA. The results are shown in FIG. 14. The amount of soluble-Flt-1 secreted from the cell sheet was significantly increased when cultured by the method of the present invention compared to plate culture. Furthermore, while no secretion of TIMP-3 was detected in plate culture, secretion of TIMP-3 was detected from the cell sheet cultured and produced by the method of the present invention. This indicates the possibility that the cell sheet produced by the method of the present invention can suppress the development of choroidal neovascularization.

### Example 6

### Production of mesenchymal stem cells multilayered sheets

A cell sheet (single layer) of mesenchymal stem cells was produced on the membrane in the same manner as in Example 1. Furthermore, new mesenchymal stem cells were seeded on the produced cell sheet at the same concentration and cultured to produce a multilayered sheet. The results are shown in FIG. 15. It was confirmed from the color tone that the multilayered cell sheet was thicker than the single layer cell sheet on the left.

### Example 7

### Evaluation of migration of cells from mesenchymal stem cell sheet

Using human adipose tissue-derived mesenchymal stem cells, cell migration was evaluated for a cell suspension in which cells were suspended, and a cell sheet produced from the cells according to the present invention. The cell migration was measured by determining whether cells inside a cell culture insert passed through a membrane and migrated to the outside of the insert using Cell Counting Kit-8 (Dojindo, Cat. CK04) and Crystal Violet Solution, 1% (SIGMA, Cat. V5265-250ML). The pore size of the membrane was 8 µm. As a cell attractant, bovine serum albumin was added at 20% or 40%.

Cell suspension: Cell attractant (or control) and basal medium were added to the cell culture plate (well and cell culture insert), and then the cell suspension was seeded on the insert. After 24 hours, the cells in the insert were removed and the number of cells in the well was evaluated. The control was not added with attractant.

Cell sheet: The cell sheet produced by the method of the present invention was used for evaluation while being held in the first culture vessel (insert). After the medium in the insert containing the cell sheet and the medium in the cell culture plate (well) were replaced with basal medium for acclimatization, the medium in the insert was replaced with basal medium, and the medium in the cell culture plate (well) was replaced with basal medium containing a cell attractant (or control). After 24 hours, the cells in the insert were removed, and the number of cells in the well was evaluated.

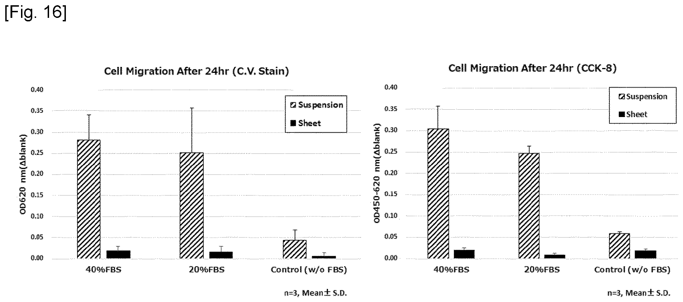

The evaluation was performed for each group (n=3). The cell count was evaluated using Cell Counting Kit-8 (Dojindo) and 1% crystal violet solution (SIGMA). The results are shown in FIG. 16. While cell migration was observed in the cell suspension, no cell migration was observed in the cell sheet produced by the method of the present invention.

The above detailed description is merely illustrative of the objects and subject matter of the present invention and is not intended to limit the scope of the appended claims. Various modifications and substitutions to the described embodiments will be apparent to those skilled in the art from the teachings set forth in the present specification without departing from the scope of the appended claims.

This application is based on Japanese Patent Application No. 2022-73877 (filing date: April 27, 2022) and Japanese Patent Application No. 2023-34995 (filing date: March 7, 2023) both filed in Japan, and the contents of both are incorporated in their entirety in the present specification.

### [Industrial Applicability]

The present invention provides a simple method for producing a cell sheet, which allows easy handling of the cell sheet produced by the method. The present invention is useful for producing a cell sheet applicable to various cells including MSCs and fibroblasts, which are cells of skin tissue, and the cell sheet produced by the method of the present invention can be used as a therapeutic implant material, and for various applications such as screening applications and toxicity testing applications.

## Claims

1. A method for producing a cell sheet, comprising the steps of:
(1) preparing one or more types of mammal-derived cells;
(2) seeding the prepared cells on a porous membrane of a first cell culture vessel having the porous membrane on the bottom thereof; and
(3) placing the first cell culture vessel into a second cell culture vessel, culturing cells, and producing a cell sheet on the porous membrane.

2. The method according to claim 1, wherein at least one of the culture medium in the first cell culture vessel or the culture medium in the second cell culture vessel is a culture medium containing serum and/or a Rho kinase (ROCK) inhibitor.

3. The method according to claim 1, wherein in the step (1), the mammal-derived cells are epithelial cells, endothelial cells, parenchymal cells or stem cells.

4. The method according to claim 3, wherein in the step (1), the mammal-derived cells are cells selected from the group consisting of pigment epithelial cells, fibroblasts, and mesenchymal stem cells.

5. The method according to claim 1, wherein in the step (2), the cells are seeded on the bottom at a density of 5,000 cells/mm² or more.

6. The method according to any one of claims 1 to 5, wherein in the cell sheet obtained by culturing, a membrane containing an extracellular matrix is formed on the side of the cells opposite to the side in contact with the porous membrane.

7. The method according to claim 6, further comprising forming tight junctions between the cells in the cell sheet obtained by culturing.

8. The method according to claim 6, further comprising the following step (4):
(4) a step of confirming the formation of a membrane containing an extracellular matrix on the side of the cells opposite to the side in contact with the porous membrane.

9. The method according to any one of claims 1 to 5, further comprising the following step (5):
(5) a step of confirming the presence or absence of the expression of a differentiation marker in the cultured cells obtained in the step (3).

10. The method according to any one of claims 1 to 5, further comprising the following step (5'):
(5') a step of confirming whether the cultured cells obtained in the step (3) secrete Soluble-Flt-1 (VEGF receptor 1) and/or TIMP-3.

11. The method according to any one of claim 1 to 4, wherein in the step (1), the mammal-derived cells are retinal pigment epithelial cells, iris pigment epithelial cells, mesenchymal stem cells or fibroblasts, and the cells are seeded on the bottom at 5,000 cells/mm² to 40,000 cells/mm².

12. A cell sheet produced by the method according to any one od claims 1 to 5.

13. An implant material for disease treatment, comprising a cell sheet produced by the method according to any one of claims 1 to 5.

14. A method for producing a membrane containing extracellular matrix, comprising a step of separating a membrane containing extracellular matrix formed on a cell sheet produced by the method according to any one of claims 1 to 5.

15. A cell sheet of mammal-derived cells arranged on a porous membrane, in which an extracellular matrix is present between the cells of the cell sheet, and a membrane containing the extracellular matrix is formed on the side opposite to the side in contact with the porous membrane.

16. The cell sheet according to claim 15, further comprising tight junctions formed between the cells.

17. The cell sheet according to claim 15, which is obtained by seeding one or more types of mammal-derived cells onto a porous membrane of a first cell culture vessel having the porous membrane on its bottom, and placing the first cell culture vessel in a second cell culture vessel and culturing.

18. The cell sheet according to claim 17, wherein at least one of the culture medium in the first cell culture vessel and the culture medium in the second cell culture vessel is a culture medium containing serum and/or a Rho kinase (ROCK) inhibitor.

19. The cell sheet according to claim 17 or 18, wherein the mammal-derived cells are epithelial cells, endothelial cells, parenchymal cells or stem cells.

20. The cell sheet according to claim 19, wherein the mammal-derived cells are cells selected from the group consisting of pigment epithelial cells, fibroblasts, and mesenchymal stem cells.

21. The cell sheet according to claim 19, wherein the mammal-derived cells are retinal pigment epithelial cells or iris pigment epithelial cells.

22. The cell sheet according to claim 19, wherein the cells contained in the cell sheet express at least one molecular marker selected from the group consisting of bestrophin-1, RPE-65, pan-Cytokeratin and any subtype of Cytokeratin, occludin, ZO-1, elastin, actin, type 1 collagen, type 2 collagen, and type 4 collagen.

23. The cell sheet according to claim 19, wherein the cells contained in the cell sheet secrete Soluble-Flt-1 (VEGF receptor 1) and/or TIMP-3.

24. A method for producing a multilayered cell sheet, comprising the following steps (1) to (4):
(1) preparing one or more types of mammal-derived cells;
(2) seeding the prepared cells on a porous membrane of a first cell culture vessel having the porous membrane on the bottom thereof;
(3) further placing the first cell culture vessel in a second cell culture vessel, culturing cells, and producing a cell sheet on the porous membrane; and
(4) repeating the following steps (a) and (b) one or more times:
(a) seeding one or more types of mammal-derived cells onto a cell sheet formed in a first cell culture vessel; and
(b) placing the first cell culture vessel into a second cell culture vessel and culturing cells to produce a new cell sheet on the cell sheet.

25. A multilayered cell sheet produced by the method according to claim 24.

26. A multilayered cell sheet arranged on a porous membrane, wherein in each cell sheet contained in the multilayered cell sheet, an extracellular matrix is present between the cells, and a membrane containing the extracellular matrix is formed on the side opposite to the side in contact with the porous membrane.
